# EUROPEAN PATENT APPLICATION

(11) **EP 2 732 814 A1**
(43) Date of publication of application: **21.05.2014**
(21) Application number: 12192991.3
(22) Date of filing: 16.11.2012
(51) Int. Cl.: A61K 9/70, A61K 9/00, A61K 9/127

(54) **Integrating reconstituted membrane proteins into free-standing planar lipid bilayers**

(71) Applicant: Paul Scherrer Institut, 5232 Villigen (CH)
(72) Inventor: Tiefenauer, Louis, 5312 Döttingen (CH); Hutter-Imhof, Ingrid, 3900 Brigerbad (CH); Demarche, Sophie, 8004 Zürich (CH)
(74) Representative: Fischer, Michael

(57) **Abstract**

A method is presented to integrate reconstituted membrane proteins in liposomes into free-standing planar lipid bilayers used for bio-analytical applications: charged proteoliposomes spontaneously fuse with preformed lipid bilayers of the opposite charge at 37 °C. The preformed bilayer can be generated by self-assembly of lipids dissolved in organic solutions or by fusion of charged liposomes to oppositely charged polymers present in micro- or nanopores.

## Description

The present invention relates to a method for fusing proteoliposomes to a preformed free-standing lipid bilayer membrane.

Membrane proteins are of pivotal importance as gate keepers of cell membranes. They regulate transport of biological building blocks such as aminoacids and nutrients (e.g. glucose), and are responsible for the transmission of signals from outside, induced by binding of hormones and potentially of drugs. Therefore, research on structure and function of the about 7500 different membrane proteins of eukaryotic cells is in the focus of biosciences. Membrane proteins need a lipid bilayer membrane to retain their full functionality. Membrane proteins are only functional in a natural environment. In case of an enforced translation of a membrane protein of interest many copies are present in the cell membrane, i.e. they are over-expressed. Such cells also can be used for the investigation of potential drug compounds.

Many classes of membrane proteins are highly relevant as drug targets. Most important are ion channels, transporters and GPCRs. Membrane proteins are found from bacterial cells to differentiated mammalian cells. Recently, the 3D-structures of a few prokaryotic and even less eukaryotic membrane proteins have been solved at an atomic resolution. The mechanisms of ion transfer by ion channels, the molecule transfer by transporters and the signal transfer by conformational changes in the transmembrane chains of GPCRs have been investigated. Further, investigations of the complex aspects of functionality, i.e. ligand binding, induced conformation changes, regulation by protein interaction and signal transfer need much more research. The atomic structure of a membrane protein of interest is a prerequisite for the understanding of its function. However, functional assays for a quantitative measurement of the mentioned functional steps will become equally important. For a detailed analysis of the working mechanism of a membrane protein e.g. the down-regulation of activity induced by binding of a drug compound at the allosteric site, purified membrane proteins are needed.

The preparation of functional and purified membrane proteins from cells includes several steps: (1) Over-expression by pro- or eukaryotic cells, (2) purification and solubilization of the membrane protein and (3) reconstitution into a lipid membrane i.e. formation of proteoliposomes. These steps have to be optimized for each membrane protein separately. The critical issue is the transfer of the fragile functional membrane protein solubilized by a suitable detergent into a lipid membrane. Integrated in a proteoliposome, the membrane protein is stabilized. Such proteoliposome suspensions can be handled like reagent solutions and are stable at least for some days at 4°C.

Membrane protein activities can be measured as optical or electrochemical signals upon addition of effectors such as natural hormones or drugs. Among the various optical detection methods fluorimetry is the most important, due to its high sensitivity and specificity and because many different fluorophores are available. For instance, fluorimetric activity detection for measuring the translocation of fluorescent effectors across membranes will allow us to assess drug resistance which is directly related to the export of antibiotics by membrane transporters such as P-glycoprotein (P-gp). Electrochemical measurements of ion channels are very common. Using specific cells with over-expressed ion channels, patch-clamp technology has been developed in the last 25 years to high throughput automated systems. Patch-clamp technologies are used to measure ion currents of ligand-gated, voltage-gated or mechano-sensitive ion channels at a predefined potential in presence or absence of compounds of interest. Ion flow across membranes can be measured using quite simple electrochemical methods, which are attractive for activity monitoring whenever electrogenic (charged) compounds are involved. Activity measurements of membrane proteins reconstituted in liposomes suffer from the fact that the internal volume is not directly accessible. Even when translocation rates of compounds from the solution into the femto-liter inner volume of a liposome are slow, as in the case of transporters, equilibrium is achieved within seconds and the ion flow stops. Ion channels have about a factor of thousand higher translocation rate and saturation is achieved even faster. Activation induced by effector compounds results in an ion flow, which is monitored electrochemically over time by inserting an electrode into a liposome or by aspiration of a single proteoliposome in a micropore present in a support. In both cases precise positioning and a high sealing are required. Optical measurements of the internal volume of liposomes by quantitative fluorescence techniques allow quantification of translocation activities.

Free-standing planar lipid bilayers separating two freely accessible compartments are attractive alternatives to liposomes. Membrane proteins integrated in such bilayers can be investigated using optical and electrochemical methods. The advantage of such planar systems is the fact, that the protein density can be defined to a certain degree and, after addition of ligands or interacting proteins to one side of the membrane, the induced activity change can be monitored over time avoiding fast saturation. However, the preparation of stable lipid bilayer membranes, in which functional membrane proteins are integrated, is not simple. It has been demonstrated that the lipid composition has an influence on the activity of the membrane proteins. Most important lipids are phosphatidyl cholines (PC) such as palmitoyloleoyl-phosphatidyl choline (POPC), which are zwitterionic. That means they have one negative charge from the phosphate group and one from the tertiary amine of the choline-residue and thus have a formal zero net charge. The uncharged steroid cholesterol makes bilayers more rigid and more uncommon lipids like ceramides have been reported to be necessary fusogens in some cases. Negatively charged lipids such as phosphatidyl-serine (PS) and positively charged phospatidyl-ethanolamines (PE) are natural triglycerides and also form bilayers.

Painting is a well-established method to achieve stable planar lipid bilayers without proteins. Lipids dissolved in an organic solvent like decane are added to a pore in an aqueous solution. The organic solvent, which is of lower specific density than water, separates from the lipids, which consequently self-assemble to a bilayer. Three major factors determine the stability of the resulting bilayer: pore size, surface chemistry and the nature of the lipids. It has been demonstrated that planar lipid bilayers in pore dimensions of nanometers to some micrometers are stable for days. Furthermore, natural occurring lipids such as POPC and POPE form stable enough bilayers. Therefore, rather exotic lipids such as di-phythanoyl-PC (DPhPC) often used for stable artificial bilayer preparations are not necessary. Residual organic solvents may remain in the pores and affect the activity of the later integrated membrane proteins. In order to avoid this problem the formation of a planar lipid bilayer can be triggered by adding negatively charged liposomes to a positively charged polymer present in the pore. The principle of using liposomes of opposite charges aimed at facilitating liposome-liposome fusion in suspension has been demonstrated in the past. Negatively charged liposomes spontaneously form a tight planar lipid bilayer also on surfaces at a relatively high temperature of 55 °C.

The next step, the insertion of the membrane protein of interest, is also difficult. Insertion should occur reliably and at a temperature at which membrane proteins do not lose their 3D-structure, i.e. denature. Fusion of uncharged lipids is observed at room temperature and high salt concentrations, but it occurs rather stochastically and higher protein densities in the planar bilayer cannot be achieved.

From literature it is known, that neutral or charged Giant Unilamellar Vesicles (GUVs) spontaneously open and fuse to supported lipid bilayers or directly to surfaces. The production of GUVs requires a special equipment and GUVs are prone to rupture by shear forces occurring during pipetting or transport in small channels e.g. of microfluidic systems.

It is therefore the objective of the present invention to provide a method for fusing proteoliposomes to a preformed free-standing lipid bilayer membrane, allowing the insertion of a reconstituted membrane protein of interest into a preformed planar bilayer of predefined lipid composition.

This objective is achieved according to the present invention by a method for fusion of proteoliposomes to a preformed lipid bilayer membrane, comprising the steps of:
a)providing an assay surface having one or a small number of pores;
b) forming free-standing lipid bilayer membranes in said pores;
c) oppositely charging the lipid bilayer membranes and the proteoliposomes, thereby enhancing the probability of spontaneously occurring fusion of the proteoliposomes to a planar lipid bilayer membrane.

This method therefore offers the possibility to insert a reconstituted membrane protein of interest into the preformed planar lipid bilayer. Due to the use of opposite charges the integration rate of reconstituted membrane proteins is superior over the known methods described above. Furthermore, the protein surface density can be controlled within a certain range.

In a preferred embodiment of the present invention, the proteoliposomes are composed from a mixture of lipids of a positive net charge, e.g. phosphatidyl ethanolamines and phosphatidyl cholines. Such compositions lead to a high probability of fusion and a concomitant integration of reconstituted membrane proteins in the free-standing lipid bilayer membrane.

A further preferred embodiment provides a reaction temperature in the range of 20 to 45°C, preferably in the range of blood temperature. This temperature range allows the proteins to stay within the range of their natural environment and to avoid denaturation.

During the fusion, the total net charge of the proteoliposomes may be different from zero and has the opposite sign of that of the preformed lipid bilayer membrane. Also, the preformed lipid bilayer membrane may comprise a partial content of not charged lipids such as cholesterol, wherein the net charge of the lipid composition is different from zero and has the opposite sign of that of the proteoliposomes.

In order to build up planar lipid bilayers and induce fusion with proteoliposomes, the pores may be within a microfluidic system which transports at controlled flow organic and/or aqueous fluids to the pores.

An advantageous procedure with respect to the generation of the lipid bilayer can be achieved, when the lipid bilayer membrane is realized in a form of a preformed planar free-standing lipid bilayer membrane which is generated by:
a) thinning processes and self-assembly of lipids dissolved in organic solvents, the so-called painting method; or
b) spontaneously rupture of Giant Unilamellar Vesicles (GUVs) at ambient or elevated temperature;
c) fusion of negatively charged liposomes to positively charged oligo- or polymeric molecules present in the pores.

In a further preferred embodiment of the present invention, the lipids may be replaced by other compounds of similar self-assembly properties as inherent to the lipids. This measure allows for building the free-standing lipid bilayer membrane according to the needs in an assay process.

Preferred embodiments of the present invention are hereinafter explained in more detail with reference to the following drawings:
Figure 1 illustrates schematically the significant method steps to integrate membrane proteins in stable lipid bilayers by two steps using an assay chip with a number of pores (A).
Figure 2 shows fluorescence microscopy images indicating the formation of the desired bilayer.
Figure 3 shows electrochemical impedance spectra (EIS) at different status of preparations according to the steps shown in Figure 1.
Figure 4 illustrates patch-clamp measurements after proteoliposome fusion at different negative and positive potentials.

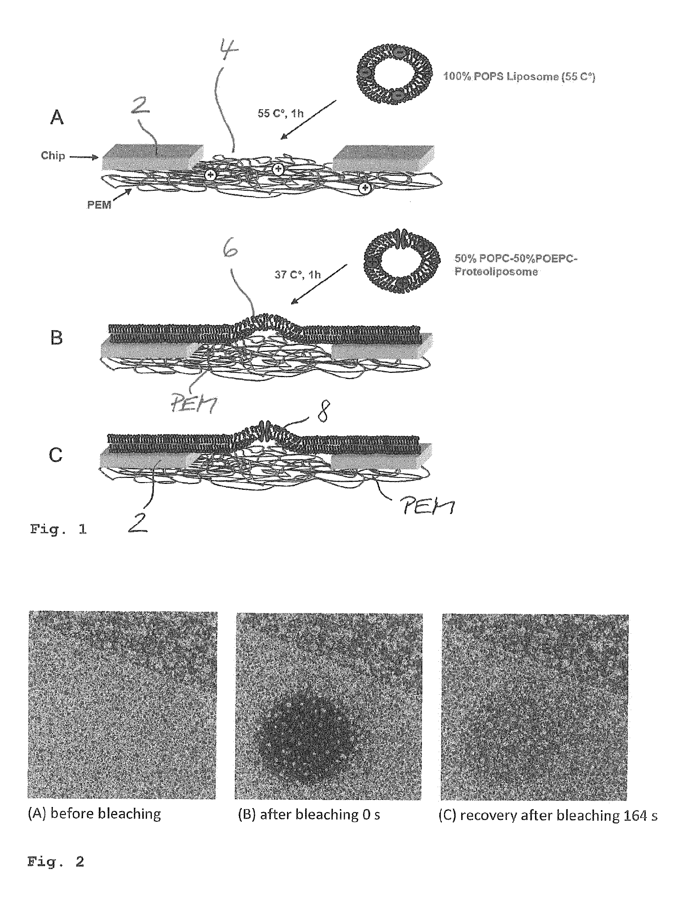

Figure 1 illustrates schematically the significant method steps to integrate membrane proteins in stable lipid bilayers by two steps using an assay chip 2 with a number of pores 4 as shown in the upper part (A). Part (A) shows also the formation of a first bilayer 6 which was formed by adding 100 µl POPS liposomes of 50 nm diameter (1 mg/ml in 10 mM HEPES with 150 mM NaCl pH 7.4) at 55 °C to a polyelectrolyte multilayer (PEM) sprayed on the back of the shown single pore 4. This polyelectrolyte multilayer PEM is positively charged in this example. The formation of the lipid bilayer 6 of negatively charged lipids by the fusion to a polyelectrolyte multilayer (PEM) is illustrated in part (B). The formation could be also achieved by painting (not shown) or other suitable method steps. As shown in part (B), the lipid bilayer 6 is fully covering the pore 4 of the assay chip 2. Therefore, the negatively charged POPS liposome form a negatively charged surface over the pore 4. Part (C) illustrates the fusion of proteoliposomes of opposite charge (here positively charged) into the preformed bilayer 6. Fusions lead to the formation of a fused bilayer 8 which now incorporates the important membrane protein the proteoliposome(s). As an example the liposomes consisting of 100 % POPS and proteoliposomes of 50% POPC/50% POEPC are shown.

Figure 2 shows three fluorescence microscopy images indicating the formation of the desired bilayer 8. For the capture of the fluorescence microscopy images, first a polyelectrolyte multilayer PEM was created on a glass slide by incubatingnegatively charged poly(Na⁺ 4-styrene sulfonate) (PSS), positively charged poly-ethyleneimine (PEI) and poly-allylimine hydrochloide (PAH) in the sequence PEI(PSS/PAH)₂PSS/PEIThe addition of 30 µl negatively charged DOPS vesicles (1mg/ml in 10 mM HEPES, 150mM NaCl pH 7.4) to the positively charged PEM surface at 55 °C resulted in the formation of a lipid bilayer , according to *Sugihara et al., 2010.* This bilayer can't be visualized by fluorescence microcopy, because no dye is present (status B of Fig.1, not shown in Figure 2).

The first microscope image (A) indicates the subsequent fusion at 37 °C of the positively charged dyed liposomes (77% POPC, 20 % POEPC and 3 % of NBD-PC (dye)) to the DOPS bilayer. By fluorescence recovery after photo-bleaching (FRAP) dyed lipids within a circle of about 20 µm diameter are bleached as shown as a dark grey area in the second image (B). The last image represents the recovery image (C) showing that dyed lipids diffuse into the bleached area. This recovery is the strong indication that fusion of the proteoliposomes to the liposome bilayer occurred.

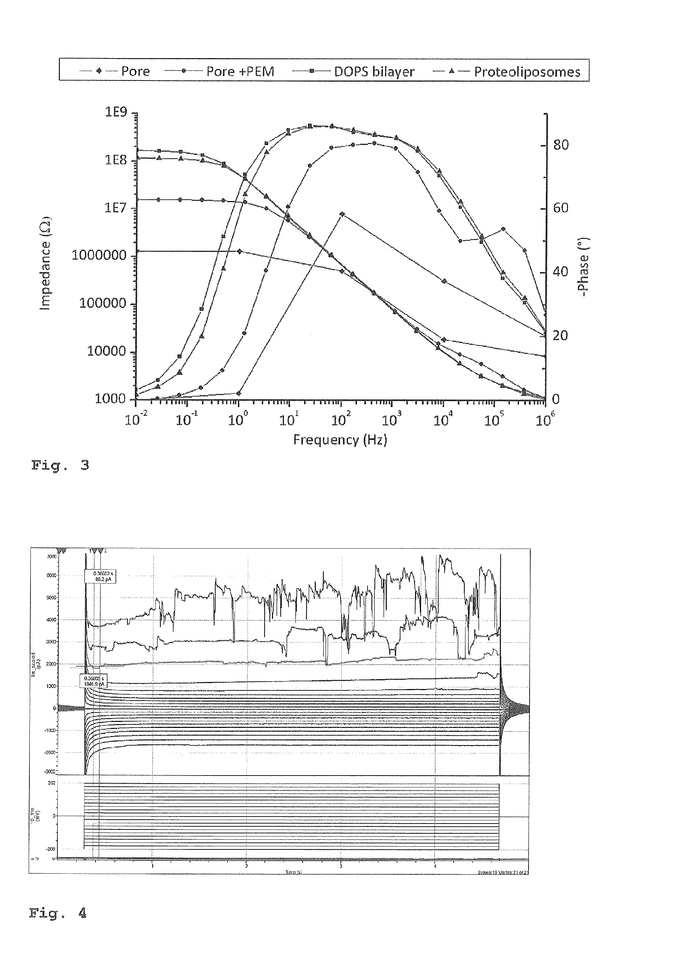

Figure 3 shows electrochemical impedance spectra (EIS) at different status of preparations according to the steps shown in Figure 1. EIS are taken from a single pore 4 of 800 nm diameter, which is present in silicon nitride membrane of 300 nm thickness (see *Han* et *al.* 2007). The course of the impedance for just the pure pore is illustrated by the symbols (◆). The course represented by the symbols (•) illustrates the situation where the pore has been filled with PEM as described in *Sugihara et al., 2010* (• = pore + PEM). After the incubation of 100 µl of a liposome suspension (100% DOPS, 1 mg/ml 10 mM HEPES 150 mM NaCl 7.4) a bilayer is formed and the impedance increases above 10⁸Ω which course is represented by the symbol (■); (■ = pore + PEM + DOPS-bilayer). After incubation of 100µl of positively charged (20%POEPC/80%POPC) proteoliposomes (details see Fig. 4), fusion to the preformed DOPS-bilayer occurs at 37 °C and the impedance decreases slightly (▲ = pore + PEM + DOPS bilayer and fused proteoliposomes).

Figure 4 illustrates patch-clamp measurements after proteoliposome fusion at different negative and positive potentials. The fusion of positively charged (20%POEPC/80%POPC) proteoliposomes (protein to lipid ratio 1:20 (w/w), 100 nm diameter) was achieved by pipetting about 100 µl of the proteoliposome suspension (1 mg/ml lipid in 10 mM HEPES with 150 mM KaCl pH 7.4) at 37 °C to the preformed negatively charged DOPS-bilayer. The preparation, reconstitution and patch-clamp measurement of the ion channel protein was described in *Hutter et al. 2012.*

### References

Sugihara, K.; Vörös, J.; Zambelli, T., A Gigaseal obtained with a self-assembled long-lifetime lipid bilayer on a single polyelectrolyte multilayer-filled manopore. ACS Nano 2010, 4, (9), 5047-5054.

Hutter, I.; Müller, E.; Kristiansen, P. M.; Kresak, S.; Tiefenauer, L., Polymer-based microfluidic device for measuring membrane protein activities. Microfluidics & Nanofluidics 2012**,** DOI: 10.1007/s10404-012-1061-0.

Han, X.; Studer, A.; Sehr, H.; Geissbühler, I.; DiBerardino, M.; Winkler, F. K.; Tiefenauer, L., Nanopore arrays for stable and functional free-standing lipid bilayers. Adv. Mater. 2007, 19, 4466-4470.

## Claims

1. A method for fusing proteoliposomes to a preformed lipid bilayer membrane, comprising the steps of:
a) providing an assay surface having one or an array of pores;
b) forming free-standing, planar lipid bilayer membranes in said pores;
c) oppositely charging the lipid bilayer membranes and the proteoliposomes, thereby facilitating spontaneously occurring fusion of the proteoliposomes to the lipid bilayer membrane.

2. The method according to claim 1, wherein the proteoliposomes are present in form of a mixture of lipids with a net positive charge, such as phosphatidyl ethanolamines and phosphatidyl cholines.

3. The method according to claim 1 or claim 2, wherein the reaction temperature is in the range of 20 to 45°C, preferably in the range of blood temperature.

4. The method according to any of the preceding claims, wherein the total net charge of the proteoliposomes is different from zero and has the opposite sign of that of the preformed lipid bilayer membrane.

5. The method according to claim 4, wherein the preformed lipid bilayer membrane comprises to partial content uncharged lipids, wherein the net charge of a lipid composition is different from zero and has the opposite sign of that of the proteoliposomes.

6. The method according to any of the preceding claims, wherein the pores are within a microfluidic system which transports at controlled flow organic and/or aqueous fluids to the pores.

7. The method according to any of the preceding claims, wherein the lipid bilayer membrane is realized in a form of a preformed free-standing lipid bilayer membrane which is generated by:
a. self-assembly and thinning processes of lipids dissolved in organic solvents, the so-called painting method; or
b. spontaneously rupture of Giant Unilamellar Vesicles (GUVs) at ambient or elevated temperature
c. fusion of negatively charged liposomes to positively charged oligo- or polymeric molecules present in the pores.

8. The method according to any of the preceding claims, wherein lipids are replaced by other compounds of similar self-assembly properties as inherent to the lipids.
